# EUROPÄISCHE PATENTSCHRIFT

(11) **EP 1 876 953 B1**
(45) Veröffentlichungstag und Bekanntmachung des Hinweises auf die Patenterteilung: **07.09.2011**
(21) Anmeldenummer: 05804610.3
(22) Anmeldetag: 17.11.2005
(51) Int. Cl.: A61B 6/00, H05G 1/02

(54) **RÖNTGENGERÄT MIT EINEM STÄNDER UND MIT EINEM AM STÄNDER IN DER HÖHE VERSCHIEBBAREN AUSLEGERARM**
X-RAY DEVICE PROVIDED WITH A COLUMN AND AN ARM UPWARDLY DISPLACEABLE THEREALONG
APPAREIL RADIOGRAPHIQUE COMPORTANT UN MONTANT ET UN BRAS POUVANT SE DEPLACER EN HAUTEUR SUR LE MONTANT

(30) Priorität: 19.04.2005 WO PCT/EP2005/051727
(43) Veröffentlichungstag der Anmeldung: 16.01.2008
(73) Patentinhaber: Swissray International Inc., Elizabeth - 07201 (VG)
(72) Erfinder: LAUPPER, Rudolf G., CH-6285 Hitzkirch (CH)
(74) Vertreter: Hepp Wenger Ryffel AG
(86) Internationale Anmeldenummer: PCT/EP2005/056027
(87) Internationale Veröffentlichungsnummer: WO 2006/111210

(56) Entgegenhaltungen:
- ES-A1- 2 152 794
- US-A- 5 469 492
- US-A- 5 901 200
- US-A1- 2002 126 470

## Beschreibung

Die Erfindung betrifft ein Röntgengerät mit einem Ständer und mit einem am Ständer in er Höhe verschiebbaren Auslegerarm gemäss dem Oberbegriff von Anspruch 1. Derartige Röntgengeräte werden heute zum Erzeugen von Röntgenbildern bzw. von Echtzeitröntgenfilmsequenzen für verschiedene Diagnosezwecke eingesetzt. Der Auslegerarm kann beispielsweise ein C-Arm sein, welcher unterschiedliche Positionierungen an liegenden, stehenden oder sitzenden Patienten ermöglicht. Der Röntgenbilddetektor ist heute zumeist zur Erzeugung digitaler Röntgenbilder vorgesehen.

Die US 5 469 492 beschreibt eine Anordnung für die Montage eines vertikal und horizontal verschiebbaren Röntgengeräts. Diese Anordnung weist einen an die Wand montierbaren Rahmen aus einem Paar horizontal beabstandeten Schienen und einem oder mehreren horizontalen Querträgern auf. An den vertikalen Schienen ist ein verschiebbarer Schlitten montiert. Auf dem Rahmen können dekorative Elemente befestigt werden, um die mechanischen Teile der Vorrichtung zu tarnen.

Die US 5 901 200 zeigt ein Röntgengerät mit einer mobilen Basiseinheit, an der ein höhenverstellbarer und drehbarer Auslegerarm geführt ist. Auf der Oberfläche dieser Basiseinheit ist ein Bildschirm auf einer lösbaren Trägerplatte befestigt.

Röntgengeräte sind zumeist zentral in einem Raum aufgestellt, wobei der Ständer einen Blickfang bildet. Es besteht daher das Bedürfnis, die Säule als Informationsträger für Bilder oder Zeichen einzusetzen. Dabei kann es sich um wechselnde Patienteninformationen, um Werbebotschaften oder einfach nur um dekorative Elemente handeln. Bei gattungsmässig vergleichbaren konventionellen Röntgengeräten lässt sich der Ständer allerdings nicht für derartige Zwecke verwenden. Es ist daher eine Aufgabe der Erfindung, ein Röntgengerät zu schaffen, bei dem auf einfache Weise, möglichst grossflächig und vorzugsweise austauschbar grafische Darstellungen am Ständer fixiert werden können. Eine weitere Aufgabe der Erfindung besteht darin, die Führung und den Getriebemechanismus für die Verschiebung des Auslegerarms so auszubilden, dass am Ständer ein möglichst grosser, zusammenhängender Flächenabschnitt verbleibt. Ausserdem soll die Zugänglichkeit zu den im Inneren des Ständers angeordneten Bauteilen verbessert werden.

Diese Aufgabe wird erfindungsgemäss mit einem Röntgengerät gelöst, das die Merkmale im Anspruch 1 aufweist. Das Aufnahmemittel am Ständer ermöglicht die Aufnahme eines Bildträgers auf einem bestimmten Flächenabschnitt des Ständers. Dabei kann es sich um eine Ebene oder auch um eine konkav oder konvex gewölbte Fläche handeln. Das Aufnahmemittel ist dabei eine am Ständer fixierbare Halterung für die vorzugsweise austauschbare Aufnahme des Bildträgers. Die Halterung kann dabei eine Art Bilderrahmen bilden, in den nach der Art eines Wechselrahmens der Bildträger einschiebbar oder einklemmbar ist.

Besonders vorteilhaft weist die Halterung zum Schutz des Bildträgers eine Abdeckung aus transparentem Material auf. Die Abdeckung kann beispielsweise aus Acrylglas oder aus Quarzglas bestehen. In bestimmten Fällen wäre es denkbar, die eine Seite der Abdeckung unmittelbar als Bildträger zu verwenden.

Zur Erzielung eines besonderen Effektes kann die Halterung eine Beleuchtung zum Beleuchten des Bildträgers aufweisen. Als besonders vorteilhaft haben sich Leuchtdioden (LED) erwiesen. Diese können im Randbereich der Halterung angeordnet sein, sodass sie seitlich Licht in die Halterung einstrahlen. Das Licht der Leuchtdioden kann dabei seitlich in eine Diffusorplatte einspeisbar sein, welche hinter dem Bilderträger angeordnet ist. Die Diffusorplatte verteilt das Licht über die gesamte Bildfläche. Der Beleuchtungseffekt kann weiter noch dadurch verbessert werden, dass auf der vom Bildträger abgewandten Seite der Diffusorplatte eine Reflektorplatte angeordnet ist. Die Reflektorplatte wirft das in der Diffusorplatte verteilte Licht nach vorne auf den Bildträger.

Die Halterung könnte aber auch einen Bildschirm oder eine Anzeige zum wechselnden Darstellen von Bildern oder Zeichen enthalten. So wäre es etwa denkbar, einen Flachbildschirm oder eine Flüssigkristallanzeige in die Halterung zu integrieren. Auf diese Weise können auch rasch wechselnde Bildsequenzen bzw. Filmsequenzen dargestellt werden. Dies könnte es beispielsweise einem Patienten ermöglichen, von ihm erstellte Röntgenaufnahmen während der Aufnahme selber zu betrachten.

Die Halterung kann sich im Wesentlichen über die gesamte Breite und Höhe des Ständers erstrecken, so dass praktisch eine ganze Seitenfront des Ständers einen Bildträger aufnehmen kann.

Eine besonders vorteilhafte Präsentation des Bildträgers wird dadurch erreicht, dass der Auslegerarm einen Führungsschlitten aufweist, der den Flächenabschnitt übergreift und der auf der dem Bildträger abgewandten Seite vorzugsweise hinter dem Aufnahmemittel am Ständer geführt ist. Mit dieser Art von Führung steht die gesamte Fläche des Flächenabschnitts zur Verfügung, ohne störende Schlitze, Führungsschienen oder dergleichen. Es wird ausserdem eine stabilere und ruhigere Führung erreicht, die auch im Zusammenhang mit konventionellen Röntgengeräten ohne Aufnahmemittel für die Aufnahme eines Bildträgers vorteilhaft sein könnte.

Der Führungsschlitten kann dabei an zwei parallelen Führungsschienen geführt sein, die jedoch vom Bildträger verdeckt sind und damit von aussen her nicht in Erscheinung treten. Die Führung an den Führungsschienen könnte dabei über Gleitlager oder über Wälzlager erfolgen.

Für die Verschiebung des Auslegerarms am Ständer kann am Führungsschlitten eine Zahnstange angeordnet sein, die mit einem am Ständer gelagerten und durch einen Antriebsmotor antreibbares Zahnritzel kämmt. Diese Antriebsart hat den Vorteil, dass weniger Masse beschleunigt und verzögert werden muss, da der Antriebsmotor fest am Ständer angeflanscht ist. Die Zahnstange kann in der Länge so dimensioniert werden, dass sie jeweils in der untersten und in der obersten Position des Auslegerarms gerade noch im Eingriff mit dem Zahnritzel ist. Das Zahnstangen-/Zahnritzel-Getriebe ist dabei vorteilhaft zwischen den beiden Führungsschienen angeordnet. Ausserdem ist es zweckmässig, wenn die Zahnstange im Eingriffsbereich des Zahnritzels auf der Rückseite abgestützt ist, um eine Durchbiegung insbesondere im Bereich der Endpositionen zu vermeiden.

Der Führungsschlitten kann ausserdem seitliche Führungswangen aufweisen, welche mit einer hinter dem Bildträger verschiebbaren Führungsplatte verbunden sind. Diese Führungsplatte kann Gleitschuhe aufweisen, die auf den beiden parallelen Führungsschienen geführt sind. So können beispielsweise pro Führungsschiene zwei im Abstand zueinander angeordnete Gleitschuhe vorgesehen sein. Die Last des Auslegerarms wird so möglichst gleichmässig auf die Führungen verteilt.

Ein Vorteil wird dadurch erzielt, dass der Auslegerarm derart aus einer Betriebsstellung in eine Ausbaustellung seitlich wegschwenkbar am Schlitten befestigt ist, dass in der Ausbaustellung die Halterung etwa im rechten Winkel zur Bewegungsebene des Schlittens ausbaubar ist. Der Auslegerarm kann zu diesem Zweck auf einer Scharnierplatte befestigt sein, welche als Bestandteil des Führungsschlittens im Abstand zum Bildträger und parallel zu diesem verschiebbar ist und welche einseitig mit Scharnieren für die Schwenkbewegung versehen ist.

Der Ständer selbst kann ein etwa quaderförmiges Gehäuse aufweisen, wobei der Bildträger bzw. die Halterung als Gehäuseabdeckung ausgebildet ist. Auf diese Weise erfüllt der Bildträger ersichtlicherweise eine doppelte Funktion. Einerseits dient er dekorativen Zwecken und andererseits erleichtert er die Zugänglichkeit zu den Bauteilen innerhalb des Ständers um Wartungs-und Reparaturarbeiten ausführen zu können.

Beim Bildträger selbst kann es sich um völlig unterschiedliche Materialien oder Konstruktionen handeln. Denkbar wäre beispielsweise eine ebene Platte aus Metall, Kunststoff, Holz, Karton oder aus einem anderen Werkstoff, welche auf einer Seite mit einer graphischen Darstellung versehen ist. Diese kann durch verschiedene Drucktechniken, fotomechanische Verfahren oder dergleichen aufgebracht werden. Beim Bildträger könnte es sich aber auch um einen Rahmen handeln, der beispielsweise mit einem textilen oder mit einem anderen Flächengebilde bespannt ist. Der Bildträger könnte auch eine reliefartige Oberflächenstruktur aufweisen oder er könnte derart aufgebaut sein, dass spezielle optische Effekte erzielt werden, wie z.B. der Effekt einer dreidimensionalen Bilddarstellung. Der Bildträger könnte schliesslich auch ein mit Flüssigkeit oder mit einem Gas gefülltes, flaches Behältnis sein, wobei spezielle Farbeffekte, Leuchteffekte oder dergleichen erzielt werden können.

Wenn der Bildträger ein Werbeträger ist, kann der Ständer des Röntgengeräts als Litfasssäule eingesetzt werden, auf welcher verschiedene Werbebotschaften dargestellt werden können. Ein derartiges Röntgengerät eignet sich beispielsweise für den Einsatz von Sponsoring als Marketingmassnahme im medizinischen Bereich. Die Finanzierung oder Mitfinanzierung eines Röntgengeräts durch einen Sponsor kann auf besonders einfache Weise auf dem Bildträger dargestellt werden.

Weitere Vorteile und Einzelmerkmale der Erfindung ergeben sich aus den Zeichnungen und aus der nachfolgenden Beschreibung eines Ausführungsbeispiels. Es zeigen:
- Figur 1: eine Ansicht eines Röntgengeräts mit Ständer und Aus- legerarm,
- Figur 2: der Bereich der Führung des Auslegerarms am Ständer in vergrösserter Darstellung,
- Figur 3: eine Draufsicht auf den Ständer gemäss Figur 2,
- Figur 4: eine perspektivische Darstellung der Getriebeanordnung für die Höhenverstellung des Auslegerarms,
- Figur 5: eine Rückansicht aus Pfeilrichtung c der Anordnung ge- mäss Figur 4,
- Figur 6: ein Querschnitt durch eine Halterung mit Bildträger,
- Figur 7: eine perspektivische Darstellung eines Röntgengeräts mit ausgeschwenktem Auslegerarm und mit ausgebauter Halterung,
- Figur 8: das Röntgengerät gemäss Figur 7 mit eingebauter Halte- rung,
- Figur 9: das Röntgengerät gemäss Figur 8 mit Auslegerarm in Be- triebsstellung,
- Figur 10: eine perspektivische Darstellung des Röntgengeräts ge- mäss Figur 9 aus einer anderen Perspektive,
- Figur 11: ein Querschnitt durch ein alternatives Ausführungsbei- spiel einer Halterung mit Bildträger, und
- Figur 12a/ Figur 12b: Bespiele von alternativen Bilddarstellung.

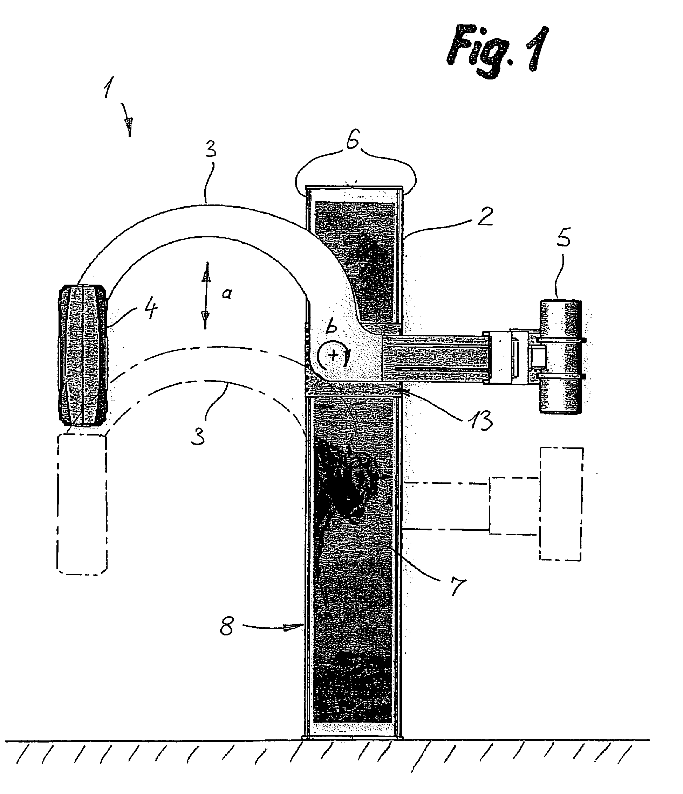

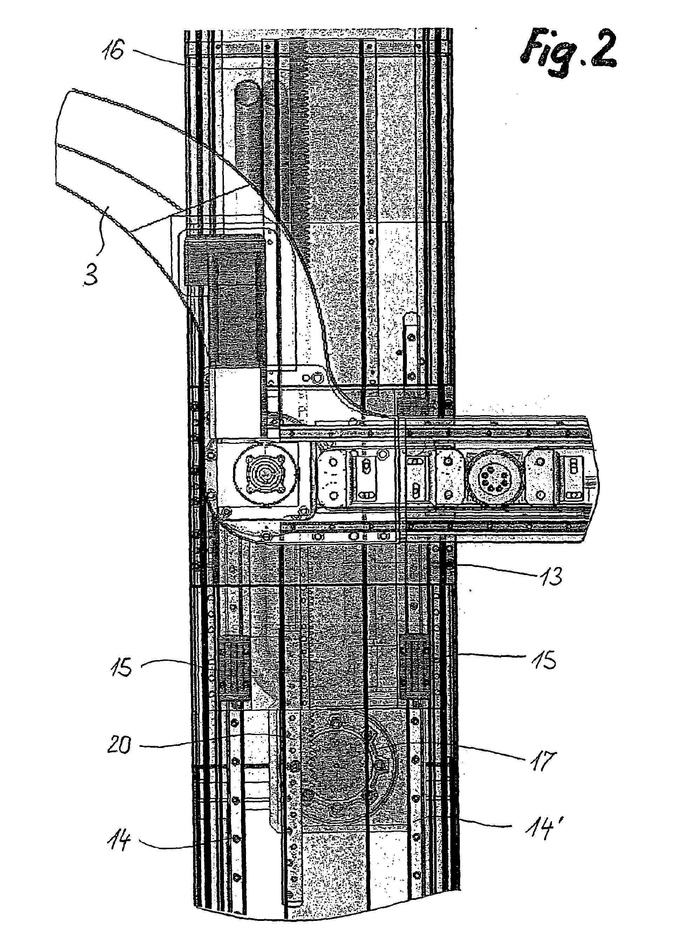

Gemäss Figur 1 besteht ein Röntgengerät 1 im Wesentlichen aus einem Ständer 2 und einem am Ständer in Pfeilrichtung a höhenverstellbar gelagerten Auslegerarm 3. Beim vorliegenden Ausführungsbeispiel handelt es sich um einen C-förmigen Auslegerarm mit einem geraden Abschnitt, wobei am Ende des C-Bogens ein Röntgenbilddetektor 4 z.B. ein Bucky und am Ende des geraden Abschnitts ein Röntgenstrahlensender 5 angeordnet ist. Der Auslegerarm 3 ist auch noch um eine horizontale Achse in Pfeilrichtung b schwenkbar. Die Führung des Auslegerarms erfolgt mit Hilfe eines Führungsschlittens 13, dessen Einzelheiten anhand der Figuren 2 bis 5 nachstehend noch beschrieben werden.

Der Ständer 2 weist Aufnahmemittel 6 zur Aufnahme eines Bildträgers 7 auf einem Flächenabschnitt 8 auf. Beim vorliegenden Ausführungsbeispiel enthält der Bildträger 7 eine Darstellung aus einem Gemälde, welche praktisch die gesamte Breite und die gesamte Höhe des Ständers 2 bedeckt.

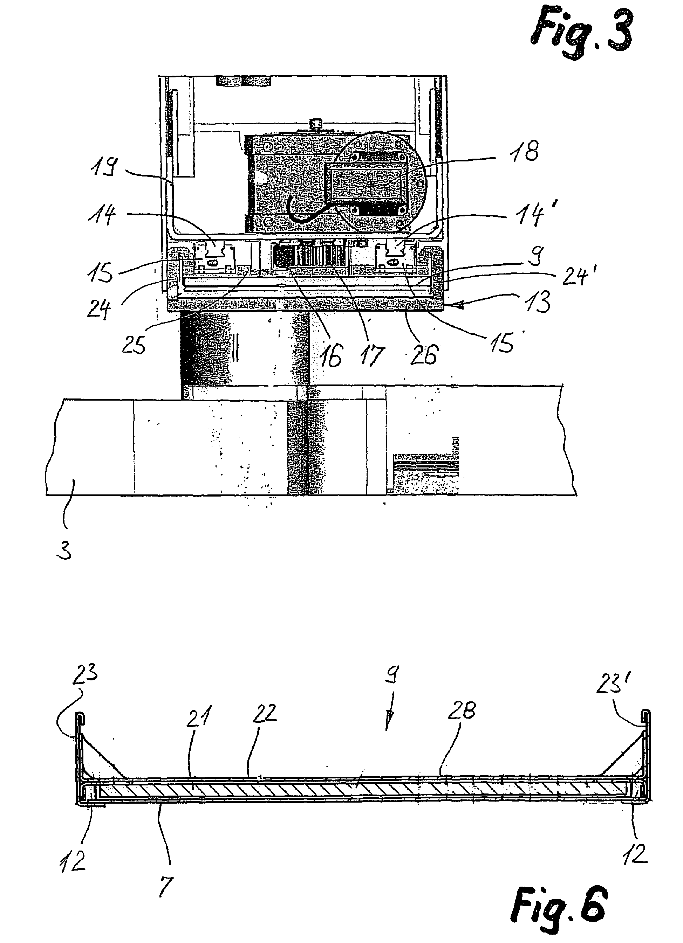

Wie aus den Figuren 2 und 3 ersichtlich ist, wird der Führungsschlitten 13 an zwei parallelen Führungsschienen 14, 14' geführt, die an einem Gehäuse 19 des Ständers angeordnet sind. Die Führung erfolgt dabei an Gleitschuhen 15, von denen je zwei pro Führungsschiene vorgesehen sind. Die Führungsschienen bzw. die Gleitschuhe sind derart konfiguriert, dass die Gleitschuhe unverlierbar und möglichst spielfrei auf den Führungsschienen gleiten.

Das Aufnahmemittel für den Bildträger besteht im Wesentlichen aus einer Halterung 9, welche am Gehäuse 19 des Ständers lediglich am unteren und am oberen Ende gehalten ist. Seitlich wird die Halterung 9 umfasst vom Führungsschlitten 13, bzw. von den Führungswangen 24 und 24'. Auch das Getriebe für die Verschiebung des Auslegerarms 3 wird auf diese Weise von der Halterung 9 vollständig abgedeckt.

Die beiden im Querschnitt J-förmigen Führungswangen 24, 24' sind mit einer Führungsplatte 25 verbunden, an welcher auch die Gleitschuhe 15 befestigt sind. Auf der Aussenseite sind die beiden Führungswangen 24, 24' mittels einer Scharnierplatte 26 verbunden, die auf einer Seite über ein Scharnier 27 an der Führungswange 24' angelenkt ist. An der Führungswange 24 ist die Scharnierplatte 26 lösbar verschraubt. An der Scharnierplatte 26 ist der Auslegerarm 3 befestigt.

Das Getriebe besteht aus einer Zahnstange 16, welche dem Schlitten 13 zugeordnet ist. Diese Zahnstange kämmt mit einem Zahnritzel 17, das auf der Aussenseite am Gehäuse 19 gelagert ist. Das Zahnritzel ist mit einem Antriebsmotor 18 im Innern des Gehäuses 19 antreibbar. Um eine durch Querkräfte verursachte Durchbiegung der Zahnstange zu vermeiden, ist diese im Bereich des Eingriffs mit dem Zahnritzel durch eine Stützleiste 20 abgestützt. Diese Stützleiste ist fest angeordnet und daher wie die Führungsschienen 14, 14' dem Gehäuse 19 zugeordnet. Um die Reibung an der Stützleiste 20 zu reduzieren, können Walzlager eingebaut sein.

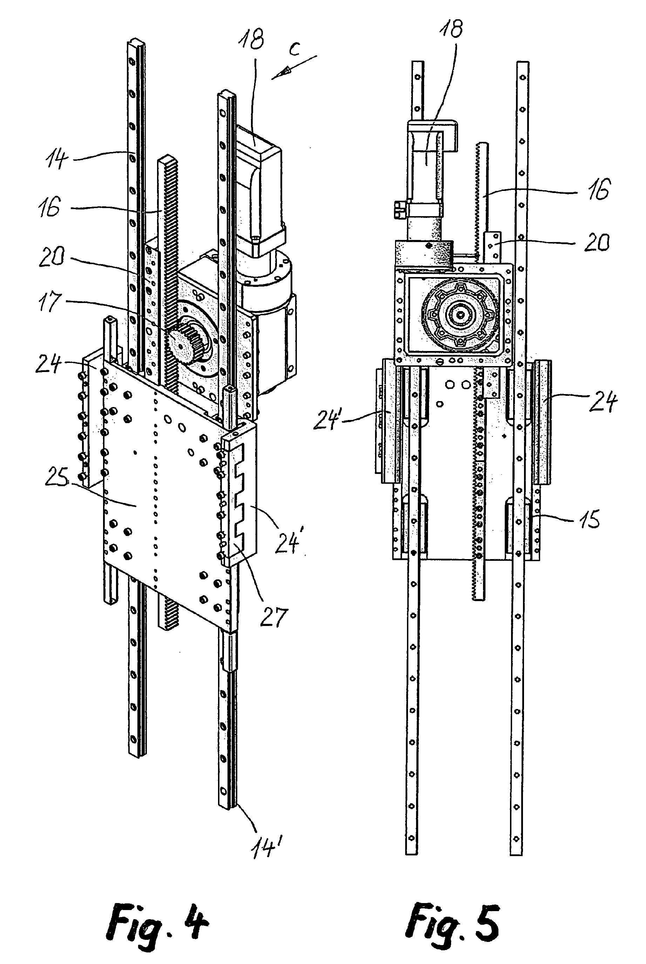

Die Figuren 4 und 5 zeigen nochmals losgelöst die gesamte Führungs- und Getriebeanordnung. Die Scharnierplatte ist hier jedoch nicht dargestellt, sondern nur das Scharnier 27. Die einzelnen Bauteile des Schlittens sind jeweils miteinander verschraubt.

Figur 6 zeigt einen Querschnitt durch die gemäss Figur 3 eingebaute Halterung 9. Die Konstruktion aus Stahlblech oder aus Kunststoff verfügt über Seitenteile 23, 23', welch sich über die gesamte Höhe des Ständers erstrecken und welche von den beiden J-förmigen Führungswangen 24, 24' umfasst werden. Die rahmenartige Konstruktion verfügt über eine Rückwand 28, auf welcher eine Reflektorplatte 22 angeordnet ist. Auf der Reflektorplatte ist eine Diffusorplatte 21 befestigt, welche das seitlich von den Leuchtdioden 12 eingespeiste Licht über die gesamte Fläche verteilt. Der Bildträger 7 beispielsweise aus einer Acrylglasplatte ist über der Diffusorplatte 21 eingeschoben und kann bei ausgebauter Halterung leicht ausgetauscht werden. Das eigentliche Bild ist als Folie auf die Rückseite des Bildträgers 7 geklebt.

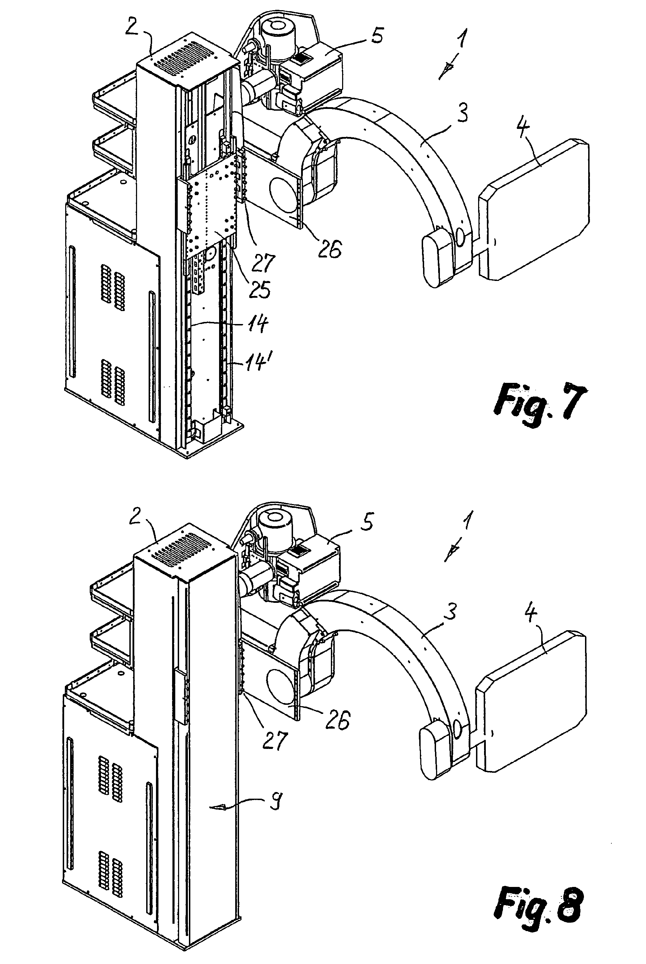

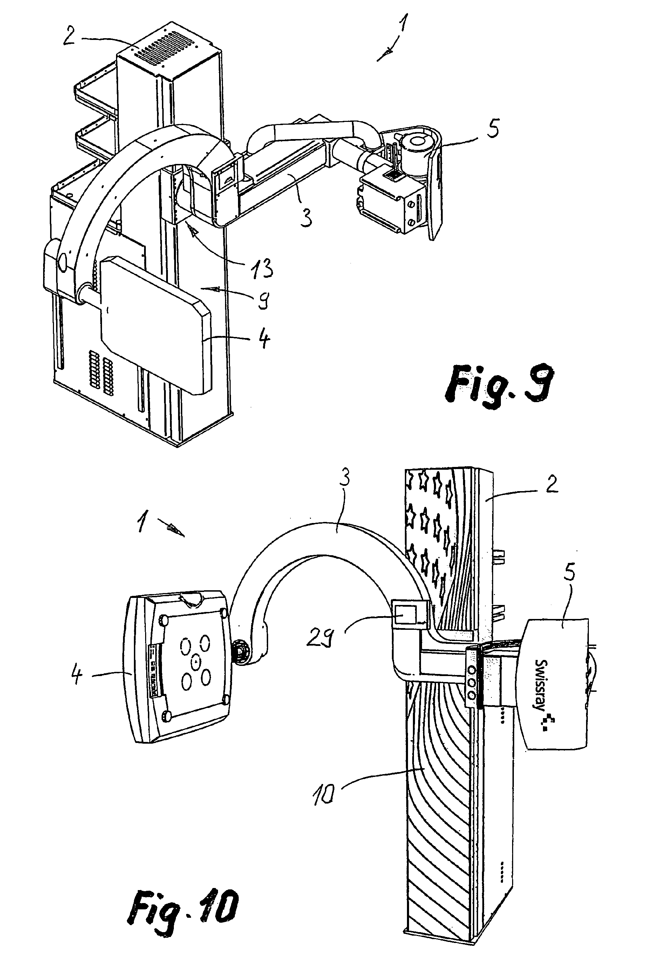

Gemäss Figur 7 ist der Auslegerarm 3 an der Scharnierplatte 26 um ca. 90° aufgeschwenkt. In dieser Stellung kann die mit dem Bildträger versehene Halterung 9 eingebaut werden, was in Figur 8 dargestellt ist. Anschliessend wird gemäss Figur 9 der Auslegerarm 3 wieder in die Betriebsstellung zurück geschwenkt und die Scharnierplatte 27 wird verschraubt.

Figur 10 zeigt das Röntgengerät aus einer anderen Perspektive mit einem Ausschnitt aus der US-Flagge als eigentliches Bild 10. Ebenfalls sichtbar ist ein Monitor 29, der am Auslegerarm 3 befestigt ist und der beispielsweise Patientendaten anzeigt.

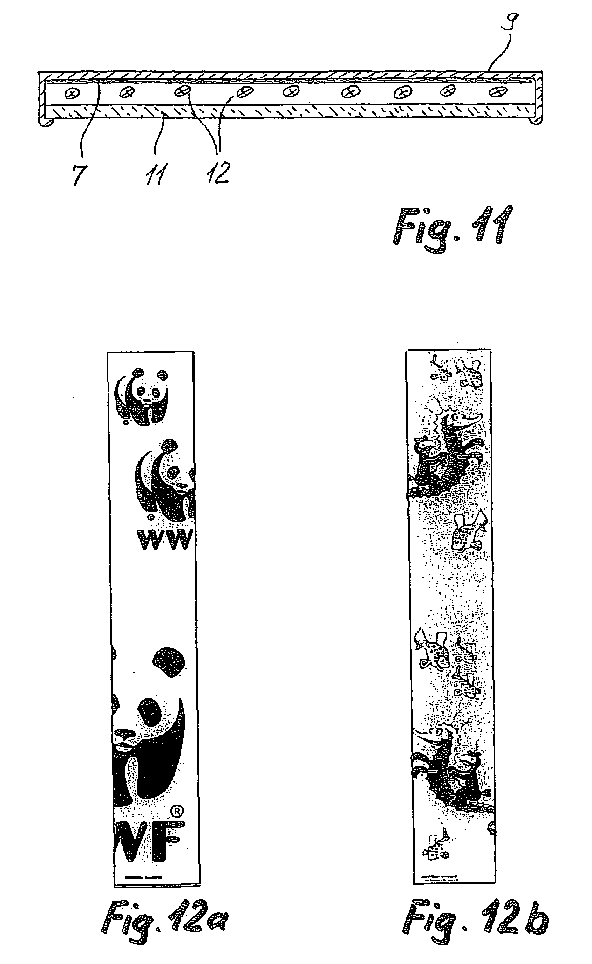

Beim Ausführungsbeispiel gemäss Figur 11 ist der Bildträger 7 unmittelbar auf der Rückwand der Halterung 9 befestigt. Eine Abdeckung 11 aus Acrylglas schützt den Bildträger und die Ausleuchtung erfolgt durch Leuchtdioden 12 oder durch andere Leuchtmittel am oberen und am unteren Ende der Halterung. Auch hier wäre es allerdings denkbar, dass die Rückseite oder die Vorderseite der Abdeckung 11 unmittelbar die Funktion des Bildträgers übernimmt. So könnte beispielsweise die Abbildung mit Hilfe eines Plotters auf eine transparente, selbstklebende Folie gedruckt werden, welche direkt auf die Oberfläche der Acrylglasplatte geklebt wird. In einem derartigen Fall dient die Rückwand der Halterung 9 als Reflektor für die Beleuchtung.

Grundsätzlich können die Leuchtmittel so angesteuert werden, dass der Bildträger stufenlos bis zum strahlenden Erleuchten dargestellt werden kann. Auch wechselnde Farben oder wechselnde Beleuchtungsintervalle lassen sich insbesondere mit Leuchtdioden sehr leicht realisieren.

Die Figuren 12a und 12b zeigen alternative Bilder 10, welche in die beschriebenen Halterungen eingesetzt werden könnten. Ersichtlicherweise wird je nach Bildauswahl ein vollständig anderer optischer Effekt erzielt, ohne dass am Gerät selbst konstruktiv irgendwelche Änderungen erforderlich sind.

## Patentansprüche

1. Röntgengerät (1) mit einem Ständer (2) und mit einem am Ständer in der Höhe verschiebbaren Auslegerarm (3), an dem wenigstens ein Röntgenbilderdetektor (4) und/oder ein Röntgenstrahlensender (5) angeordnet ist, sowie mit Aufnahmemittel (6) am Ständer (2) zur Aufnahme eines Bildträgers (7) auf einem Flächeabschnitt (8) des Ständers, wobei das Aufnahmemittel eine am Ständer fixierbare Halterung (9) für die Aufnahme des Bildträgers ist, **dadurch gekennzeichnet, dass** der Auslegerarm (3) einen Führungsschlitten (13) aufweist, der den Flächenabschnitt (8) übergreift und der auf der dem Bildträger abgewandten Seite am Ständer geführt ist, wobei der Auslegerarm derart aus einer Betriebsstellung in eine Ausbaustellung seitlich wegschwenkbar am Führungsschlitten (13) befestigt ist, dass in der Ausbaustellung die Halterung etwa im rechten Winkel zu Bewegungsebene des Führungsschlittens ausbaubar ist.

2. Röntgengerät nach Anspruch 1, **dadurch gekennzeichnet, dass** die Halterung zum Schutz des Bildträgers eine Abdeckung (11) aus transparentem Material aufweist.

3. Röntgengerät nach Anspruch 1 oder 2, **dadurch gekennzeichnet, dass** die Halterung eine Beleuchtung (12) zum Beleuchten des Bildträgers aufweist.

4. Röntgengerät nach Anspruch 3, **dadurch gekennzeichnet, dass** die Beleuchtung aus mehreren Leuchtdioden besteht, die sich über wenigstens einen Randbereich der Halterung erstrecken und deren Licht seitlich in eine hinter dem Bildträger angeordnete Diffusorplatte (21) einspeisbar ist.

5. Röntgengerät nach Anspruch 4, **dadurch gekennzeichnet, dass** auf der vom Bildträger abgewandten Seite der Diffusorplatte eine Reflektorplatte (22) angeordnet ist.

6. Röntgengerät nach einem der Ansprüche 1 bis 5, **dadurch gekennzeichnet, dass** die Halterung einen Bildschirm oder eine Anzeige zum wechselnden Darstellen von Bildern oder Zeichen enthält.

7. Röntgengerät nach einem der Ansprüche 1 bis 6, **dadurch gekennzeichnet, dass** sich die Halterung im Wesentlichen über die gesamte Breite und Höhe des Ständers erstreckt.

8. Röntgengerät nach einem der Ansprüche 1 bis 7, **dadurch gekennzeichnet, dass** der Führungsschlitten (13) an zwei parallelen Führungsschienen (14, 14') geführt ist.

9. Röntgengerät nach einem der Ansprüche 1 bis 8, **dadurch gekennzeichnet, dass** am Führungsschlitten (13) eine Zahnstange (16) angeordnet ist, die zum Verschieben des Führungsschlittens mit einem am Ständer (2) gelagerten und durch einen Antriebsmotor (18) antreibbaren Zahnritzel (17) kämmt.

10. Röntgengerät nach Anspruch 8 und 9, **dadurch gekennzeichnet, dass** das Zahnstangen-/Zahnritzel-Getriebe zwischen den beiden Führungsschienen (14, 14') angeordnet ist.

11. Röntgengerät nach einem der Ansprüche 1 bis 10, **dadurch gekennzeichnet, dass** der Führungsschlitten (13) seitliche Führungswangen (24, 24') aufweist, welche mit einer hinter dem Bildträger verschiebbaren Führungsplatte (25) verbunden sind.

12. Röntgengerät nach Anspruch 8 und Anspruch 11, **dadurch gekennzeichnet, dass** die Führungsplatte Gleitschuhe (15) aufweist, die auf den beiden parallelen Führungsschienen (14, 14') geführt sind.

13. Röntgengerät nach einem der Ansprüche 1 bis 12, **dadurch gekennzeichnet, dass** der Auslegerarm auf einer Scharnierplatte befestigt ist, welche als Bestandteil des Führungsschlittens im Abstand zum Bildträger und parallel zu diesem verschiebbar ist und welche einseitig mit Scharnieren für die Schwenkbewegung versehen ist.

14. Röntgengerät nach einem der Ansprüche 1 bis 13, **dadurch gekennzeichnet, dass** der Ständer ein etwa quaderförmiges Gehäuse aufweist und das der Bildträger (7) bzw. die Halterung als Gehäuseabdeckung ausgebildet ist.

15. Röntgengerät nach einem der Ansprüche 1 bis 14, **dadurch gekennzeichnet, dass** der Bildträger eine transparente Platte
ist und das Bild als Folie auf die Platte aufgeklebt ist.

16. Röntgengerät nach einem der Ansprüche 1 bis 15, **dadurch gekennzeichnet, dass** der Bildträger ein Werbeträger ist.

## Claims

1. X-ray device (1) having a column (2) and having a cantilever arm (3) whose height can be displaced on the column and on which at least one X-ray image detector (4) and/or one X-ray transmitter (5) is arranged, and having mounting means (6) on the column (2) for mounting an image carrier (7) on a surface section (8) of the column, the mounting means being a holder (9), that can be fixed on the column, for the mounting of the image carrier, **characterized in that** the cantilever arm (3) has a guide carriage (13) that overlaps the surface section (8) and that is guided on the column on the side averted from the image carrier, the cantilever arm being fastened on the guide carriage (13) in a way capable of being pivoted away laterally from an operating position into a dismantling position, this being done in such a way that in the dismantling position the holder can be dismantled approximately at right angles to the movement plane of the guide carriage.

2. X-ray device according to Claim 1, **characterized in that** the holder has a cover (11) made from transparent material for protecting the image carrier.

3. X-ray device according to Claim 1 or 2, **characterized in that** the holder has a light (12) for illuminating the image carrier.

4. X-ray device according to Claim 3, **characterized in that** the light consists of a number of light-emitting diodes that extend over at least an edge region of the holder and whose light can be fed laterally into a diffuser plate (21) arranged behind of the image carrier.

5. X-ray device according to Claim 4, **characterized in that** a reflector plate (22) is arranged on the side of the diffuser plate averted from the image carrier.

6. X-ray device according to one of Claims 1 to 5, **characterized in that** the holder includes an image screen or a display for the alternating display of images or characters.

7. X-ray device according to one of Claims 1 to 6, **characterized in that** the holder essentially extends over the entire width and height of the column.

8. X-ray device according to one of Claims 1 to 7, **characterized in that** the guide carriage (13) is guided on two parallel guide rails (14, 14').

9. X-ray device according to one of Claims 1 to 8, **characterized in that** arranged on the guide carriage (13) is a rack (16) that, for the purpose of displacing the guide carriage, meshes with a pinion (17) which is supported on the column (2) and can be driven by a drive motor (18).

10. X-ray device according to Claims 8 and 9, **characterized in that** the rack/pinion gear is arranged between the two guide rails (14, 14').

11. X-ray device according to one of Claims 1 to 10, **characterized in that** the guide carriage (13) has lateral guide cheeks (24, 24') that are connected to a guide plate (25) that can be displaced behind the image carrier.

12. X-ray device according to Claims 8 and 11, **characterized in that** the guide plate has skids (15) that are guided on the two parallel guide rails (14, 14').

13. X-ray device according to one of Claims 1 to 12, **characterized in that** the cantilever arm is fastened on a hinged plate that, as component of the guide carriage can be displaced at a distance from the image carrier and parallel thereto, and that is provided on one side with hinges for the pivoting movement.

14. X-ray device according to one of Claims 1 to 13, **characterized in that** the column has an approximately cuboidal housing, and that the image carrier (7) or the holder is designed as housing cover.

15. X-ray device according to one of Claims 1 to 14, **characterized in that** the image carrier is a transparent plate, and the image is bonded as a film onto the plate.

16. X-ray device according to one of Claims 1 to 15, **characterized in that** the image carrier is an advertising medium.

## Revendications

1. Appareil radiographique (1) comprenant un support (2) et un bras (3) pouvant coulisser en hauteur sur le support, bras sur lequel sont disposés au moins un détecteur d'images radiographiques (4) et/ou un émetteur de rayons X (5), et des moyens d'enregistrement (6) sur le support (2) pour l'enregistrement d'un support d'image (7) sur une partie de surface (8) du support, le moyen d'enregistrement étant une attache (9) pouvant être fixée sur le support pour l'enregistrement du support d'image, **caractérisé en ce que** le bras (3) présente un chariot de guidage (13), qui recouvre la partie de surface (8) et qui est guidé sur le côté opposé au support d'image sur le support, le bras étant fixé sur le chariot de guidage (13) de façon à pouvoir basculer latéralement d'une position de service dans une position de démontage, de telle sorte que, dans la position de démontage, l'attache puisse être démontée à peu près à angle droit par rapport au plan de déplacement du chariot de guidage.

2. Appareil radiographique selon la revendication 1, **caractérisé en ce que** l'attache présente un revêtement (11) à base de matériau transparent pour la protection du support d'image.

3. Appareil radiographique selon la revendication 1 ou 2, **caractérisé en ce que** l'attache présente un éclairage (12) pour l'éclairage du support d'image.

4. Appareil radiographique selon la revendication 3, **caractérisé en ce que** l'éclairage est constitué de plusieurs diodes électroluminescentes, qui s'étendent sur au moins une zone périphérique de l'attache et dont la lumière peut être injectée latéralement dans une plaque de diffuseur (21) disposée derrière le support d'image.

5. Appareil radiographique selon la revendication 4, **caractérisé en ce qu'**une plaque de réflecteur (22) est disposée sur le côté, opposé au support d'image, de la plaque de diffuseur.

6. Appareil radiographique selon l'une quelconque des revendications 1 à 5, **caractérisé en ce que** l'attache contient un écran ou un affichage pour la représentation alternative d'images ou de caractères.

7. Appareil radiographique selon l'une quelconque des revendications 1 à 6, **caractérisé en ce que** l'attache s'étend principalement sur toute la largeur et toute la hauteur du support.

8. Appareil radiographique selon l'une quelconque des revendications 1 à 7, **caractérisé en ce que** le chariot de guidage (13) est guidé sur deux rails de guidage (14, 14') parallèles.

9. Appareil radiographique selon l'une quelconque des revendications 1 à 8, **caractérisé en ce qu'**une crémaillère (16), qui, pour le déplacement du chariot de guidage, s'engrène avec un pignon denté (17) monté sur le support (2) et pouvant être entraîné par un moteur d'entraînement (18), est disposée sur le chariot de guidage (13).

10. Appareil radiographique selon les revendications 8 et 9, **caractérisé en ce que** la transmission crémaillère/pignon denté est disposée entre les deux rails de guidage (14, 14').

11. Appareil radiographique selon l'une quelconque des revendications 1 à 10, **caractérisé en ce que** le chariot de guidage (13) présente des joues de guidage (24, 24') latérales, qui sont reliées à une plaque de guidage (25) pouvant coulisser derrière le support d'image.

12. Appareil radiographique selon la revendication 8 et la revendication 11, **caractérisé en ce que** la plaque de guidage présente des patins (15), qui sont guidés sur les deux rails de guidage (14, 14') parallèles.

13. Appareil radiographique selon l'une quelconque des revendications 1 12, **caractérisé en ce que** le bras est fixé sur une plaque à charnière, qui, en tant qu'élément constitutif du chariot de guidage, peut coulisser à distance du support d'image et parallèlement à celui-ci et qui est dotée sur un côté de charnières pour le mouvement de basculement.

14. Appareil radiographique selon l'une quelconque des revendications 1 à 13, **caractérisé en ce que** le support présente un boîtier de forme à peu près parallélépipédique et **en ce que** le support d'image (7) ou l'attache est conçu (e) comme revêtement de boîtier.

15. Appareil radiographique selon l'une quelconque des revendications 1 à 14, **caractérisé en ce que** le support d'image est une plaque transparente et l'image est collée comme une feuille sur la plaque.

16. Appareil radiographique selon l'une quelconque des revendications 1 à 15, **caractérisé en ce que** le support d'image est un support publicitaire.
